# EUROPEAN PATENT APPLICATION

(11) **EP 4 578 291 A1**
(43) Date of publication of application: **02.07.2025**
(21) Application number: 24223584.4
(22) Date of filing: 29.12.2024
(51) Int. Cl.: A23C 11/10, A23L 2/38, A23L 11/60, A23L 11/65, A23L 33/105, G01N 33/02, G01N 33/14, A23B 2/42, A23B 11/13, A23B 11/137, A23B 75/00, A23B 70/30, G01N 33/18

(54) **METHOD AND SYSTEM FOR PRODUCING A LIQUID FOOD PRODUCT**

(30) Priority: 29.12.2023 EP 23220751
(71) Applicant: Tetra Laval Holdings & Finance S.A., 1009 Pully (CH)
(72) Inventor: BONNEAU, Paul, 92800 Puteaux (FR); BARR, Justin, 22186 Lund (SE)
(74) Representative: Tetra Pak - Patent Attorneys SE

(57) **Abstract**

The disclosure relates to a method (200) of producing a liquid food product (LP), the method (200) comprising
mixing (201) water (W) and a food material (F) to provide a mixture (M),
heat treating (202) the mixture (M) for deactivating harmful microorganisms,
evaporating (203) water from the mixture (M), to provide a stream of water vapor (WV), and a stream of liquid that forms the liquid food product (LP),
condensing (204) the water vapour (WV) to form a condensate (WC),
measuring (205) an ammonium content (A-r1) in at least one of the mixture (M), the condensate (WC) and the liquid food product (LP),
determining (206) an ammonium content value (A-r2) that represents the measured ammonium content, and
determining (207) whether the ammonium content value (A-r2) exceeds a threshold (ΔA-n) that is based on a nominal ammonium content value (A-n). The disclosure also relates to a system.

## Description

### Field of invention

The invention relates to a method of producing a liquid food product. The invention also relates to a system for producing a liquid food product.

### Background

Plant-based liquid food products are growing in popularity in recent years. To enhance the shelf-life of such products, it is commonly known to use different types of systems for providing heat treatment of the liquid food in the food processing industry, also known as ultra-high temperature (UHT) systems. These systems are typically used for sterilizing the liquid food, as well as making the liquid food ready for distribution efficiently and safely in relation to requirements related to the food processing industry.

Commonly, within all food production, tests may be performed on the end product to ensure the quality of the food, such as the product's smell, taste, and/or colour. If the test indicates that the product does not fulfil the quality requirements, a large batch of the product may need to be discarded or recycled in the process. This implies large costs and ineffective production.

One reason for unsatisfactory product quality can be related to bacterial growth in the system and thus initiation of a cleaning process of the production system is needed. The cleaning processes of a food production system are often set to be performed at a specific time interval or between two batches.

Moreover, the sustainability requirements for production processes are becoming stricter each year, and producing companies are forced to take more responsibility for their production and the waste that they produce.

Thus, there is still a need for a production method and system solution that fulfils the requirements related to the food processing industry, while at the same time ensuring good product quality and minimising product that goes to waste or have to be recirculated into the process.

### Summary

It is an object of the invention to provide a method and a system addressing at least some of the problems and fulfilling the need of a method that ensures good product quality.

This object has been achieved by a method of producing a liquid food product, the method comprising
mixing water and a food material to provide a mixture,
heat treating the mixture) for deactivating harmful microorganisms,
evaporating water from the mixture, to provide a stream of water vapor, and a stream of liquid that forms the liquid food product,
condensing the water vapour to form a condensate,
measuring an ammonium content in at least one of the mixture, the condensate and the liquid food product,
determining an ammonium content value that represents the measured ammonium content, and
determining whether the ammonium content value exceeds a threshold that is based on a nominal ammonium content value.

This object has also been achieved by a system for producing a liquid food product, the system comprising
a mixing arrangement configured to mix water and a food material to provide a mixture,
a heat treatment arrangement configured to heat treat the mixture (M) for deactivating harmful microorganisms,
a flash vessel or an evaporator configured to evaporate water from the mixture, to provide a stream of water vapor, and a stream of liquid that forms the liquid food product,
a condenser configured to condense the water vapour to form a condensate,
a control unit comprising software instructions for
   measuring an ammonium content in at least one of the mixture, the condensate and the liquid food product,
   determining an ammonium content value that represents the measured ammonium content, and
   determining whether the ammonium content value exceeds a threshold that is based on a nominal ammonium content value.

Some food materials, such as oat and soy, naturally includes certain amounts of ammonia or chemical compounds that naturally may give rise to ammonia. In the process of producing plant-based beverages, the food material is mixed with water, and enzymes may be added into the mixture to hydrolyse the plant-based food material to cut starch and create sugars. In the process of producing the plant-based beverages, substances within the food material may induce a chemical reaction with the enzymes, which are added, or present in the specific food material, when they are all mixed with water. In some cases, these chemical reactions result in the creation of ammonia. As an example, glutamine, which is present in oat and soybeans, may react with the enzymes added such that the glutamine and water reacts together with the enzymes such that the product is glutamate and ammonia. Whereby, ammonia, when dissolved in water, form ammonium hydroxide, hereafter referred to as ammonium.

As long as the ammonium level in the liquid food product, and thus also in the water condensate, is close to a nominal value, the product quality is not affected. However, if the ammonium content in the liquid food product, and consequently in the water condensate, increases or decreases such that the ammonium content value exceeds or fall below a threshold value, the product quality may be affected.

Increased levels of ammonium may be caused by: a food material that has started to ferment due to improper storage, the mixture is held within a storage tank for too long such that it starts to ferment, and/or by bacterial build-up in the production system.

Decreased levels of ammonium may be caused by, e.g., wrong dosage of enzymes in the holding stage.

An advantage of the present method, and system, is that the control of the product's end quality is enhanced. Since the ammonium content is monitored, necessary activities, to ensure high product quality, can be performed in advance, thus preventing the product from being affected and thereby minimising the risk that the finished product must be discarded, or recycled in the process, which in turn ensures a cost-efficient production.

Another advantage is that the knowledge about the waste of the production process, i.e., the water condensate, is enhanced. By measuring and controlling the waste, suitable actions may be taken to ensure that the waste is handled properly and in accordance with existing governmental restrictions.

Moreover, such a method and system are advantageous in that the ammonium content in the condensate is known, and thereby the ammonium may be recovered from the condensate to generate a by-product which may be sold.

Heat treating the mixture is of importance to partly of fully sterilize the product and deactivating, or kill, harmful microorganisms such that the produced beverage is safe to consume. The heat treatment can be done by e.g., an indirect or direct UHT-system.

The determining of an ammonium content value and the determining of whether the ammonium content value exceeds the threshold value may be performed by a control unit. The control unit may be a central processing unit, CPU, a memory unit, or any other control unit commonly known.

The control unit may receive information from at least one sensor which is configured to measure the ammonium content in at least one of the mixture, the condensate and the liquid food product.

By measuring the ammonium content in at least one of the mixture, the water condensate and the liquid food product and comparing the measured value, or a mean value of the measured values over time, with a nominal ammonium content value, the ammonium content value may be supervised. If the ammonium content value reaches undesirable values, a control signal is provided by the control unit allowing necessary actions to be made to prevent a faulty product.

It is to be noted that the term "exceeds" refers to a value representing the ammonium content value which is above or below a threshold, the threshold being e.g., range of acceptable values based on the nominal ammonium content value. Thus, exceeding the threshold refers to values being too low or too high to be within the range.

The nominal ammonium content value may be a predetermined value that is associated with the type of food product to which the liquid food product belongs. As mentioned, food materials such as oats and soy, naturally includes ammonia, and thereby includes an expected amount of ammonia, and thus the nominal ammonium content can be predetermined based on the expected value.

Alternatively, or additionally, the nominal ammonium content value may be determined by measuring the ammonium content in at least one of the mixture, the condensate and the liquid food product, during a first period of time, and determining a set of ammonium content values that represent the ammonium content measured during the first period of time, and thereafter setting the nominal ammonium content value as a mean of the determined ammonium content values. The first period of time may be a fixed, predetermined time. Alternatively, or as a complement, the first period of time may be adjusted such that a reasonable steady-state is obtained.

It is to be noted that the ammonium content may be measured by at least one sensor. The system may comprise one sensor but may alternatively comprise a plurality of sensors, e.g., a first, second, and third sensor.

The sensor may be a sensor capable of detecting ammonium in a liquid, such as a sensor with ion-selective electrodes.

It is to be noted that the heat treatment arrangement and evaporating arrangement may be an integrated system, such as a falling film evaporator, or a separate system, such as direct heating device and a flash vessel.

It is to be noted that the system may comprise any other commonly known heat treatment arrangement and/or evaporating arrangement.

In this context, the term "direct heating device" may be any device capable of increasing a temperature of a liquid or steam in an easy and efficient way, i.e., a device which is configured to heat the liquid or steam. By way of example, the direct heating device may be a steam injector or a steam infuser.

As readily appreciated by the person skilled in the art, the flash vessel should be interpreted as a vessel capable of performing flash evaporation (or partial evaporation). Flash evaporation (or partial evaporation) is the partial vapor that occurs when a saturated liquid stream, i.e. the stream, undergoes a reduction in pressure by passing through a throttling valve or other throttling device. Preferably, a part of the stream "flashes" into vapor in the flash vessel. Both the vapor and the residual of the stream are cooled to the saturation temperature of the stream at the reduced pressure.

During production, one purpose of the flash vessel is to expel water from the liquid food. Thus, when the liquid food is introduced via the direct heating device, the liquid food dilutes in the direct heating device. Having the flash vessel which is able to expel water from the liquid food, the flash vessel is advantageous as it is capable of restoring the liquid food as it was before being diluted in the direct heating device. Another purpose of the flash vessel during production is to quickly decrease the temperature of the liquid food. By way of example, the temperature of the liquid food is increased by the steam injector to approximately 140 °C and the temperature of the liquid food is decreased in the flash vessel to approximately 70-80 °C.

As readily appreciated by the person skilled in the art, the falling film evaporator should be interpreted as a device capable of evaporating fluid, such as water, from a product mixture. A falling film evaporator may advantageously be used when the liquid product is to be a condensate. An advantage of the falling film evaporator is that the product does not need to be diluted by e.g., steam, during the process of heat treatment.

Generally, all terms used in the claims are to be interpreted according to their ordinary meaning in the technical field, unless explicitly defined otherwise herein. All references to "a/an/the [element, device, component, means, step, etc]" are to be interpreted openly as referring to at least one instance of said element, device, component, means, step, etc., unless explicitly stated otherwise. The steps of any method disclosed herein do not have to be performed in the exact order disclosed, unless explicitly stated.

### Drawings

Embodiments will now be described, by way of example, with reference to the accompanying schematic drawings, in which
Fig. 1A discloses a flowchart illustrating a Fig. 1A is a flowchart illustrating a first embodiment of a system for heat treatment of liquid food.
Fig. 1B disclose a flowchart illustrating a second embodiment of a system for heat treatment of liquid food.
Fig. 2 is a flowchart illustrating steps of a method for performing producing a liquid food product and measuring the ammonium content value in the condensate.
Figs. 3-4 discloses different charts reflecting the measured ammonium content in the water condensate over time.

### Description

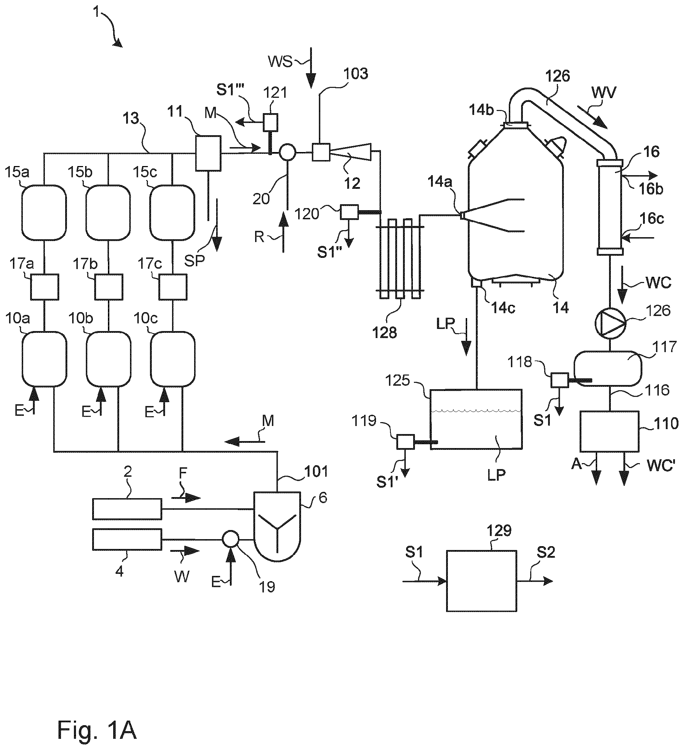

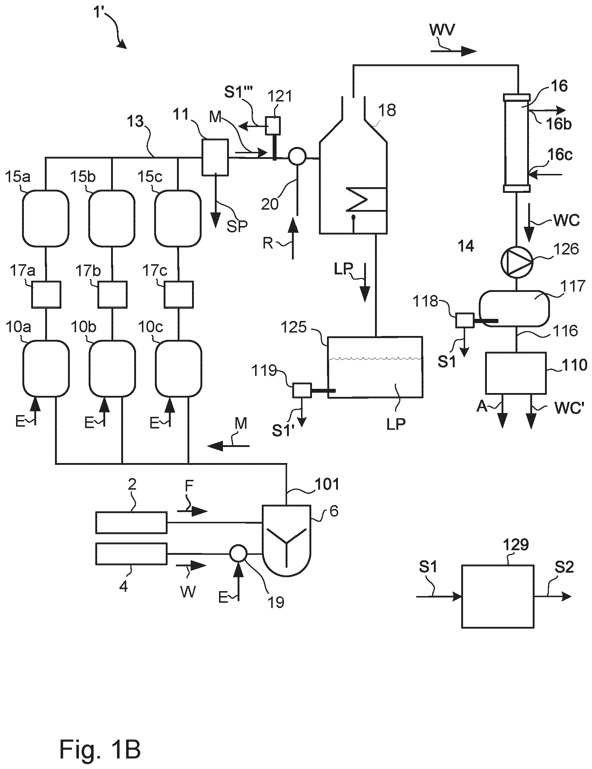

With reference to Fig. 1A and 1B, a flowchart of a system 1, 1' for heat treatment of food product mixture M is illustrated by way of example. The system 1, 1' is preferably an ultra-high temperature (UHT) system. The system 1, 1' is illustrated in a highly simplified manner in which only parts relevant for understanding how to perform measurements of the ammonium content A-r1 in the water condensate WC, in the mixture M before, or after, heat treatment, and/or in the liquid food product LP. Hence, it is to be understood that the system 1,1' typically comprises further parts not shown or described in the following disclosure.

With reference to Fig. 1A, an exemplary system 1 for producing a plant-based liquid food product LP is illustrated, in which the system 1 generally uses plant-based food material F and water W to form a mixture M in a mixer 6. The plant-based food material F may be added to the mixer 6 from a container 2, such as a hopper, which may be adapted for conveying dry goods. The plant-based food material F may include any suitable type of food material F or combination of food material for making the plant-based liquid food product LP. This includes, but is not limited to, one or more of oat, pea, soy, rice, beans or the like. The plant-based food material F may be in any suitable form when added to the mixer 6, such as a dry form, e.g., flour, flake, kernel, groat, seed, grains, pea, bean, nut, or the like, or any combination thereof. Additional ingredients may also be added to the mixer 6 to form the mixture M as may be desired to facilitate processing, taste, nutritional composition or other properties of the final liquid food product LP.

The water W may be added from a separate container or other source 4, such as a municipal water source. The water W is added to the mixer 6 as a liquid and may be heated prior to entering the mixer 6. Alternatively, or additionally, the mixture M may be heated in the mixer 6, such as with a heater in thermal communication with the mixer 6. The temperature of the water W or the mixture M in the mixer 6 may be in a range from 55 °C to 95 °C. The amount of water W added to the mixer 6 depends on the desired properties of the mixture M. For example, the mixture M may have a solids content in a range from e.g., 10% to 35%, or 16% to 20% by weight, or more, such that the amount of water W added to the mixer 6 may be in a range from 65% to 90% by weight, or less.

As seen in Figs. 1A-B, three containers 10a-c are located downstream of the mixer 6 on the line 101 and are configured to receive the mixture M and enabling the mixture M to be treated with enzymes E and/or with heat. That is, the mixture M may be either treated with enzymes, heat, or both. Heat treatment may be done by supplying heat to the containers 10a-c, for example by using a heating jacket that surrounds the containers 10a-c. The enzymes E may, if used, hydrolyze the plant-based material in the mixture M to cut starch and create sugars. Following this process, the enzymes may be deactivated, either in the containers 10a-c, or in a separate deactivation container 17a-c which is located downstream of containers 10a-c.

Enzymes E may be added to the containers 10a-c, however, enzymes E could also be added directly to the mixer 6 at an enzyme inlet 19. In such a case, the containers 10a-c may be used for subsequent processing such as deactivation. The treatment with enzymes and/or heat is performed according to known techniques commonly employed for the type of plant material that is used to form the liquid food product LP.

It is to be noted that there might be more, or less, than three containers 10a-c. Each container 10a-c may be fluidly connected with a respective deactivation container 17a-c, or each container 10a-c may be connected to one, or more, common deactivation containers 17a-c.

A holding container 15a-c is located downstream of the enzyme deactivation container 17a-c, to hold the mixture M before it is further transported into the system 1, 1'.

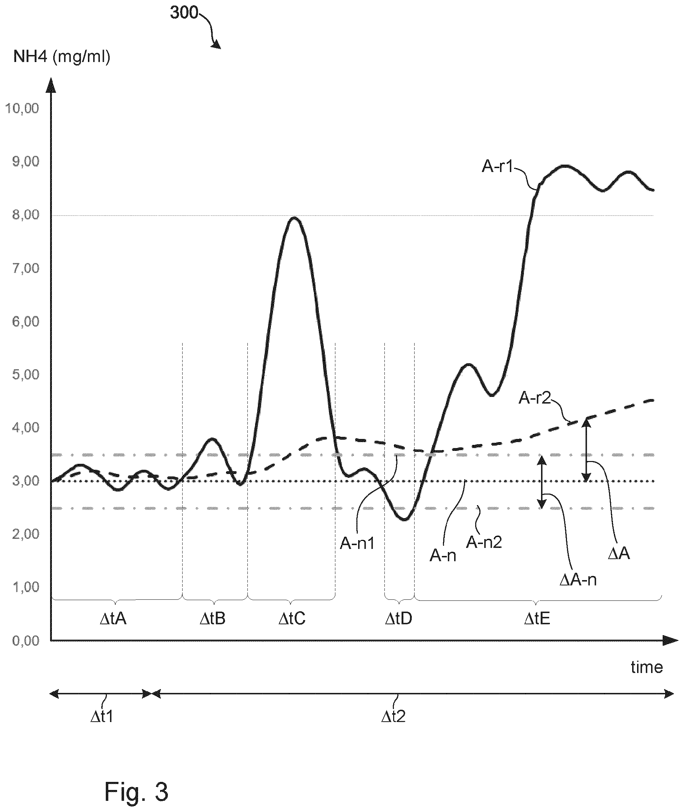

There may be one holding container 15a-c for each deactivation container 17a-c as shown in Figs. 3-4, or each deactivation container 17a-c may be connected to one or more common holding containers 15a-c. The deactivation container 17a-c and the holding container 15a-c may be one and the same.

A separation unit 11 is located downstream of the mixer 6 and is configured to receive the mixture M and separate it into a solid portion SP and a liquid portion of the mixture, of which the solid portion SP may include fibrous material separated from the plant-based material F. The solids content in the liquid portion of the mixture M may be in a range from 10% to 30% by weight, for example, with a balance being the water from the food product mixture.

The separation unit 11 may be a decanter or other suitable device. It is to be noted that the separation unit 11 may be optional, such as, when producing a hole-oat product, separation of the solid portion SP and the liquid portion is not desired and thus no separation unit 11 is present.

Downstream the separator, a first sensor 121 for measuring the ammonium content in the mixture is located. The first sensor 121 provides the control unit 129 with a signal S1‴ comprising information about the measured ammonium.

The appearance of an increased level of ammonium at this stage of the process may be caused by: fermentation of the food material F due to improper storage conditions, fermentation of the mixture M due to that the mixture M has been stored in the container 10a-c and/or the holding container 15a-c for too long, or the container 10a-c or the holding container 15a-c contain residual from an earlier batch of product, wherein the residual has fermented.

The mixture M portion is subjected, as shown in Fig. 1A, to direct heating by a direct heating device 12 which is configured to receive the mixture M via a product inlet line 13, and a water steam WS via a steam inlet line 103. The direct heating device 12 is configured to create a stream comprising a mixture of the food material mixture M and the water steam WS, and consequently, the temperature of the mixture M is increased. The temperature of the stream may be at least 130°C when entering the flash vessel 14. Thus, the direct heating device 12 itself may be capable of heating the stream to at least 130°C. Additionally, or alternatively, the direct heating device 102 and the heating device 128 may together be capable of heating the stream to at least 130°C.

The stream, containing the mixture M and water steam WS, is fed to the flash vessel 14, via the heating device 128 which is configured to increase the temperature of the stream even further prior to entering the flash vessel 14. A second sensor 120 is located in proximity to the heating device 128 to measure the ammonium in the stream and send a signal S1" to the control unit 129. Due to the increased temperatures, substances in the mixture M may decompose which can give rise to increased ammonium levels, about an 10% increase, and thus, the amount of ammonium in the stream may increase. It is to be noted that the second sensor 120 may be located after the heating device 128.

The stream enters the flash vessel 14 via a flash vessel inlet 14a. In the flash vessel 14, the stream pressure decreases, and consequently the temperature of the stream decreases, wherein the liquid food product LP is emptied from the flash vessel 14 at a bottom outlet 14c and the liquid food product enters an end product container 125. In the end product container 125, a third sensor 119 is located. The third sensor 119 measures the ammonium content in the produced liquid food product LP and provides a signal S1' to the control unit 129.

Liquid food product LP that may not adequately fulfil the quality requirements may be recycled into the process. The recycled product R is feed into the system through a recycle inlet line 20 such that the recycled product R can be heat treated once more.

A flow of water vapour WV is drawn from the flash vessel 14, via an upper outlet 14b of the flash vessel 14, to the condenser 16 where the water vapor WV is condensed to water condensate WC. The condenser 16 may be a tube heat exchanger in which a majority of the water vapor become condensate as well as the temperature of the water vapor may be decreased.

The condenser 16 comprises a fluid outlet 16b and a fluid inlet 16c.

Thereafter, the water condensate WC is drawn from the condenser 16 to a vacuum pump 126. A buffer container 117 is located downstream of the vacuum pump 126 so as to manage the flow from the vacuum pump. A fourth sensor 118 is located in the buffer container 117 to measure the ammonium content in the water condensate WC present in the buffer container 117. The sensor 118 sends a signal S1 to the control unit 129.

The control unit 129 is configured to receive signals S1, S1', S1", S1‴ from one or more sensors 118, 119, 120, 121 and to determine an ammonium content value A-r2 (see Fig. 3) that represents the measured ammonium content A-r1, and determining whether the ammonium content value A-r2 exceeds a threshold ΔA-n that is based on a nominal ammonium content value A-n. If the ammonium content value A-r2, at at least one measuring location, exceeds (falls outside of) the threshold value (threshold range) ΔA-n, a control signal S2 is provided. In Fig. 3, the difference between the ammonium content value A-r2 and the nominal ammonium content value A-n is indicated by ΔA.

Downstream of the sensor 118, along line 116, a water condensate handling arrangement 110 is located. The water condensate handling arrangement 110 may be configured to either separate the water condensate WC' and the contained ammonium A such that water condensate WC' and ammonium A can be recovered or removed separately, or be configured to separate the water condensate WC' and the contained ammonium A such that water WC' can be recovered and e.g., recycled in the process via a reintroducing line, and the ammonium A can be removed, or be configured to remove i.e., drain, the water condensate WC' together with the contained ammonium A.

The ammonium A may be extracted from the water condensate WC by any commonly known process, e.g., ammonia stripping or air stripping.

With reference to Fig. 1B, an alternative system 1' is shown. The system 1' as shown in Fig. 1B, have many similarities to the system 1 as shown in Fig. 1A. However, the alternative system 1' differs in that the heat-treating process and evaporation process is performed in an integrated system of an evaporator 18, such as a falling film evaporator. The evaporator 18 is located downstream of the separation unit 11 and is configured to receive and evaporate at least part of the mixture M to produce at least one evaporated portion in form of water vapor WV and at least one concentrated portion in form of the liquid food product LP. The water vapor WV is derived from the water W in the initial mixture M. Similar to the system 1, as shown in Fig. 1A, the system 1' comprises several sensors 118, 119, 121 which are arranged along the process to measure the ammonium content and send a signal S1, S1', S1‴ to the at least one control unit 129.

About 80% to 95%, or more, of the water in the mixture M may be evaporated into water vapour WV. The temperature of this evaporated water may be in a range from 70 °C to 90 °C. About 99% or more of the evaporated portion EP may be evaporated water, with a balance being impurities, such as ammonium.

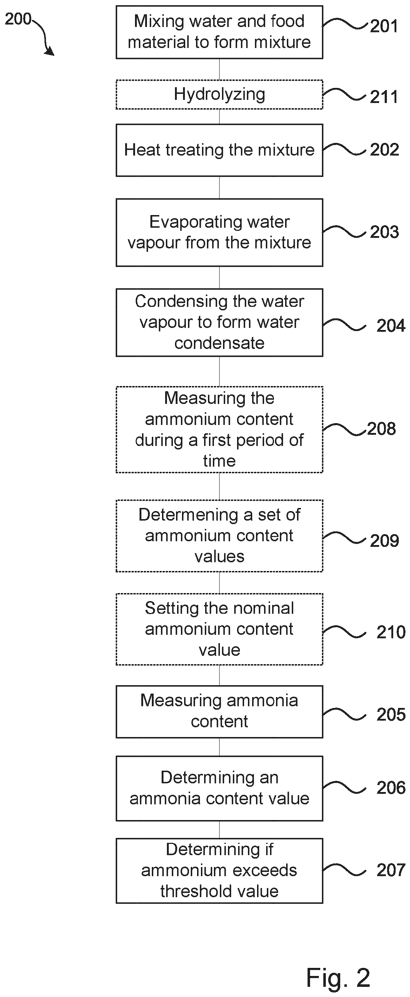

With reference to Fig. 2, a flowchart illustrating a method 200 for producing a liquid food product LP and measuring the ammonium content A-r1 in at least one of the mixture M, condensate WC, and the liquid food product LP, is shown. The method 200, as shown in Fig. 2, begins with a step of mixing 201 a at least a food material F and water W to form a mixture M. The mixture M may be prepared at an elevated temperature, such as by heating the water W or heating the food product mixture M.

The step 211 includes extracting nutrients from the food material F to the mixture M. The extracting of nutrients may be performed by treating the mixture M with enzymes E, which hydrolyze the food material F. The enzyme treatment may occur over a time span of 2, 4, 6, or even up to 15 hours. Generally, the amount of enzyme added may be less than 1% by weight of the total mixture M. Following enzyme treatment, the enzymes may be deactivated.

In step 202, the mixture M is be treated with heat, as appropriate in view of what type of plant-based liquid food product is prepared.

At step 202, the mixture M is heat treated by e.g., a direct steam injector 12 and the in step 203, the food product mixture is evaporated by e.g., a flash vessel 14 such that the liquid food product LP and water vapor WV is formed. Alternatively, step 202 and step 203 are performed simultaneously in e.g., a falling film evaporator 18.

At step 204, the water vapor WV is drawn from the evaporator 18 or flash vessel 14 to the condenser 16 to condense the water vapor WV into water condensate WC.

At step 205, the ammonium content A-r1 in at least one of the mixture M, the condensate WC, and the liquid food product LP is measured.

An ammonium content value A-r2 is determined at step 206 and represents the measured ammonium content A-r1.

It is determined in step 207 whether the ammonium content value A-r2 exceeds a threshold ΔA-n that is based on a nominal ammonium content value (A-n).

The nominal ammonium content value A-n, may, as will be described in further detail below, be based on a predetermined target value, or the method may, as shown in Fig. 2, comprise the steps of measuring 208, determining 209, and setting 210 a nominal content value A-n as a mean of the measured ammonium content A-r1 during a first period of time Δt1.

With reference to Fig. 3, an exemplified production process 300 of a soy-based liquid food product is shown as a graph. During the production process the ammonium content is measured in at least the mixture M, the water condensate and/or the liquid food product LP. It has been realized that by measuring the ammonium content value at at least one of these stages of the production process, the control of the end product quality is enhanced, and knowledge about recurring events can be provided such that preventive actions may be taken to avoid, or at least reduce, deterioration of the finished product. A preventive action may be to plan more frequent sterilizations cycles and/or reduce the dilution rate when recirculating an already produced product into a new batch.

It is to be noted that the exemplified production process 300, as shown in Fig.3, may be equally applicable to a production process of a liquid food product LP based on another type of plant-based material, e.g., oat.

The ammonium content A-r1, measured in mg/l, and shown on the Y-axis, has been measured in the water condensate WC over a period of time, and the ammonium content value A-r2 has been determined.

The production process 300, as shown in Fig. 3, extends over a time period of several hours, such as at least 3 hours, at least 10 hours, or even more.

The nominal ammonium content value A-n may also be referred to as a reference value or a target value. The nominal ammonium content value A-n may be based on: an assumption of the ammonia content associated with the specific food material F, a database specifying the ammonia content associated with the specific food material F, and/or by measuring the ammonium content A-r1 in e.g., the water condensate WC, over a first period of time Δt1 to determine a steady state of the ammonium content A-r1. The nominal value A-n of ammonium A in the water condensate WC during production of a plant-based beverage may be: 3 mg/l when the beverage is based on organic soy, 6 mg/l when based on non-organic soy, 25 mg/l when based on oat, and 1 mg/l when based on almond.

The threshold ΔA-n may be an allowable variation of the ammonium content value A-r2. The threshold ΔA-n has an upper threshold value A-n1 and a lower threshold value A-n2, wherein the threshold values A-n1, A-n2 comprises a range that is offset from the nominal ammonium content value A-n by at least 5 to 20%.

The ammonium content value A-r2 represent a mean of measured ammonium contents A-r1 during a period of time. By the term "mean" it intend to refer to one or more numbers or values that best represents a set of set of ammonium content values. It may be the arithmetic mean, geometric mean, harmonic mean, generalized mean, weighted arithmetic, or any other suitable type of mean calculated by using known methods within the fields of mathematics and statistics.

The mean value of the measured ammonium content may be used to minimize the risk of causing false alarms. The term "false alarm" refers to a situation where the alarm is provided due to an isolated exceeding of the threshold value An1, A-n2 which after a short time-period, e.g., a few minutes, returns, by itself, to an acceptable value. Such an isolated exceeding will not noticeably affect the quality of the liquid food product LP, and thus no actions need to be taken. By instead using a mean value A-r2 of the measured ammonium A-r1, an alarm can be provided when the measured and determined ammonium content value A-r2 has exceeded the threshold value A-n1, A-n2 such that an action can be taken to prevent deterioration of the product quality. However, in some processes it might be favourable and desired to obtain an alarm on the instant measured ammonium content A-r1 and not base the alarm set-up on a mean value of the measured ammonium content A-r1.

Fig. 3 illustrates different time intervals ΔtA-E which may occur during the production of a soy-based beverage. Note that these situations, which are to be described in further detail below, are equally applicable and realistic for a production process for a beverage based on another plant-base, such as oat.

Time interval ΔtA, as shown in Fig. 3, represent a normal production of a soy-based product. The ammonium A quantity is fluctuating along the production as it depends on the flow of the mixture M and water condensate WC. The upper threshold value A-n1 is set to be 3.5 mg/l and the lower threshold value A-n2 is set to be 2.5 mg/l such that the allowable mean value A-r2 is set to be between 2.5 mg/l and 3.5 mg/l. As shown in time interval ΔtA, the ammonium content value A-n2 is within the threshold ΔA-n, thus no alarm is activated. In a case where the system 1, 1' is set to provide an alarm based on the instant measured value A-r1, no alarm would be activated since the measured ammonium content A-r1, during time interval ΔtA, also is within the set threshold ΔA-n.

During time interval ΔtB, as shown in Fig. 3, there is a peak of ammonium quantity in the water condensate WC. This small peak occurs for a short period of time and may be linked to a process adaptation, food material F quality variation, increased evaporation, or quality of the water and/or steam induced in mixture M.

"Process adaptation" refers to the stage after the machines in the system 1, 1' have been sterilised and/or where changes may have been made. A process adaptation may cause temporary unbalance in the production system 1, 1' which will, after some time, be stabilized.

If the sensors, in this situation, monitor the instant measured ammonium value A-r1, the system 1, 1' will send an alarm since the ammonium content A-r1 exceeds the threshold ΔA-n. However, if the alarm is programmed to be activated when the mean value of the measured and determined ammonium content value An2 exceeds the threshold ΔA-n, the system 1, 1', in this situation, will not provide an alarm as the mean value A-r2 is within the threshold range.

As shown in Fig. 3, with reference to time interval ΔtC, a peak of the measured ammonium content A-r1 causes the ammonium content value A-r2 to exceed the threshold value A-n1. This large increase may be caused by recirculating an already produced batch of liquid food product LP. Recirculation of a liquid food product LP batch may be performed when the finished product does not pass the quality check, or when an issue with the production process arises such that there is a risk that recently produced products have a deviated quality due to e.g., insufficient sterilization. It has been realised that when a recirculation of product is performed, the ammonium content increases, and when the recirculation stops, i.e., when a new batch is introduced into the system, the ammonium content A-r1 reverts to a nominal value A-n.

The large increase may also be caused by changing of batch. Commonly, after the system 1, 1' has been cleaned and sterilised, several batches may pass the production system 1, 1' before another cleaning process is initiated. One batch generally constitutes 30 000 I of liquid food product LP, which generally represent approximately 3 hours of production. If an increase of ammonium A is detected during the cycle between two cleaning processes, and where the ammonium content A-r1 relatively quick returns to the nominal value A-n, it may be caused by the system being unbalanced due to introduction of a new batch. This will result in that a small portion of the batch will have a slightly different ammonium A concentration than the previous batch. If the ammonium content A-r1 does not return to the nominal value A-n, the liquid food product LP is spoiled either by bacterial growth in the system 1, 1', or by poor food product FP quality.

With reference to time interval ΔtD, as shown in Fig. 3, the ammonium content level A-r1 in the water condensate WC have decreased such that the value has passed below the lower threshold value A-n2. The small drop during time interval ΔtD may be linked to process adaptation, as discuses above, or decreased evaporation leading to less concentration in the water condensate. A decreased ammonium content value caused by a decreased evaporation may imply that the product is diluted.

If the system 1,1' is programmed to provide an alarm based on the instant value, the system 1, 1' will send an alarm since the measured ammonium content A-r1 is below the lower threshold value A-n2. On the other hand, if the system 1,1' is programmed to base the alarm activation on the determined ammonium content value A-r2 based on the mean value of the measured ammonium content A-r1, the system 1,1' will not provide an alarm as the mean value A-r2 is within the acceptable range ΔA-n.

With reference to time interval ΔtE, as shown in Fig. 3, a peak of measured ammonium content A-r1 is observed, and the ammonium content A-r1 does not decrease and seems to be stabilizing to a new value. It has been realized that when such a situation occurs, it is most often linked to: wrong dosage of the enzymes E, insufficient heat treatment such that the enzymes E are not deactivated, or food material F spoilage.

If the deactivation of enzymes E does not function properly, remains of enzymes E are still active and will spoil the product.

Enzymes E are most often not added in the production of a soy-based beverage since the soybean naturally contain enzymes E in its shell. Thus, wrong dosage of enzymes E is thereby most often not the cause of increased ammonium levels in a soy-based product. However, in a process related to the production of an oat-based beverage, and where a situation similar to the situation occurring during time interval ΔtE of process 300, it is most often caused by the wrong dosage of enzymes E, i.e., a too large portion of enzymes E are added, which tend to increase the ammonium content A-r1, and if the dosage is not changed during the cycle, the ammonium content A-r1 will stabilize on a new higher level.

Another reason for such a large increase of ammonium, relevant for both soy and oat, may be that the raw product, i.e., the food material F, is not stored and/or mixed under proper conditions, and/or within a certain time limit. A food material F which has been stored under wrong conditions, e.g., in a humid climate, and/or has been stored for too long may be exposed to bacterial growth which will induce an increase of ammonium.

A change of the ammonium content A-r1 within the same batch could occur in case of a production failure and/or the system 1,1' is not sterilized often enough which cause bacterial growth in the production system. A change of the ammonium contentA-r1 in between two batches may indicate that the second batch has been mixed to early such that it has been stored for too long before being heat treated, and thus such that the mix has started to ferment, and a change of the ammonium content when the raw product is changed may indicate that the plant base quality, or the mixed food product quality, is changed and does not reflect the quality of the previous batch.

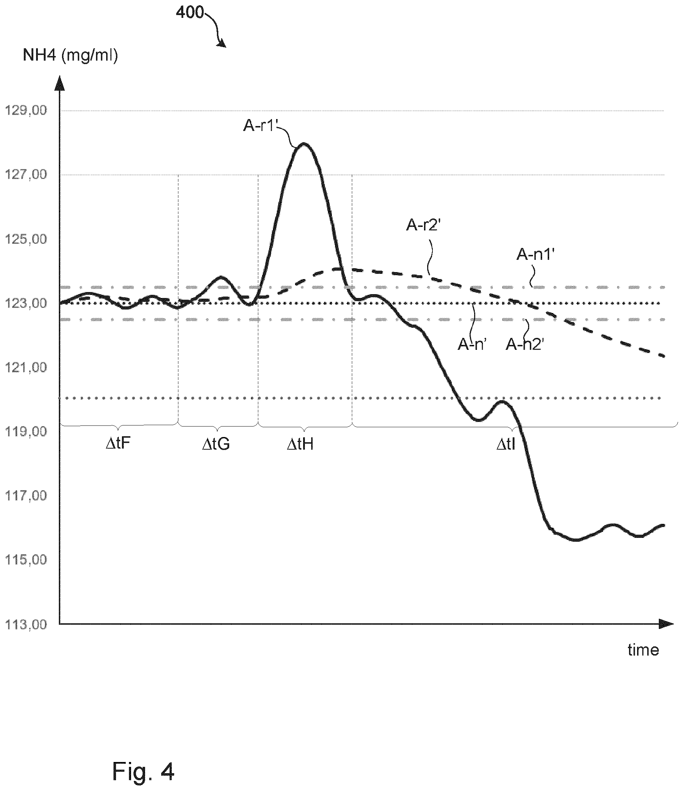

With reference to Fig. 4, an exemplified production process 400 of an oat-based liquid food product LP is shown as a graph. During time interval ΔtF, the ammonium content A-r1' in the water condensate WC is fluctuating around the nominal value A-n'. The fluctuation may depend on the flow of the food product mixture M and the water condensate WC. The measured ammonium content A-r1' exceeds the upper threshold value A-n1' during the time interval ΔtG-H, as shown in Fig. 4, this increase may be caused by the same reasons as described in relation to the increased ammonium levels A-r1 during time intervals ΔtB-C in Fig. 3.

During time interval Δtl, as shown in Fig. 4, a substantial decrease of the ammonium content A-r1' is observed and the decrease causes the ammonium content value A-r2' to also exceed the lower threshold value A-n2'. This decrease may be caused by a lack of enzymes E or that the enzymes E are missing.

In a production process, not shown, where the nominal ammonium value is based on a data set, the ammonium content value, already from the beginning of the production process, may be far above, or below, the nominal ammonium content value and the threshold value. When producing a product where enzymes E need to be added, increased or decreased values of ammonium may be caused by: enzymes E are not dosed properly, i.e., to small or to large amount of enzymes E are added, the quality of the enzymes E, the deactivation of the enzymes E is malfunctioning, the batch is damaged due to the growth of microorganisms, and/or the food material F does not fulfil the expected quality.

As shown in Figs. 3-4, the system 1, 1' will at some points provide a control signal S2 due to that the ammonium content value A-r2 has reached outside the threshold value A-n1, A-n2. When such a control signal S2 is provided there are several actions that can be made, such as: initiating a sterilization process of the production system, test the food material F, test the process water W, test the steam WS which is to be infused in the food product mixture.

An advantage of observing the ammonium content value on the system is that it may provide knowledge about recurring events such that preventive actions may be taken to avoid, or at least reduce, deterioration of the product. A preventive action may be to plan more frequent sterilizations cycles and/or reduce the dilution rate when recirculating an already produced product into a new batch.

Even though it is preferred that the system is designed in accordance with the disclosure in the detailed disclosure of preferred embodiments and the appended drawings, it should be noted that a specific preferred embodiment of a specific component does not necessarily have to be combined with a specific embodiment of another component. Thus, advantages associated with a specific embodiment, including one or more features, of a specific component may be accomplished even though the other components are designed in accordance with the more general disclosure under the summary of the invention rather than being defined in accordance with the specific embodiment disclosed in the detailed description.

Additionally, variations to the disclosed embodiments can be understood and effected by the skilled person in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage.

## Claims

1. A method (200) of producing a liquid food product (LP), the method (200) comprising
mixing (201) water (W) and a food material (F) to provide a mixture (M),
heat treating (202) the mixture (M) for deactivating harmful microorganisms,
evaporating (203) water from the mixture (M), to provide a stream of water vapor (WV), and a stream of liquid that forms the liquid food product (LP),
condensing (204) the water vapour (WV) to form a condensate (WC),
measuring (205) an ammonium content (A-r1) in at least one of the mixture (M), the condensate (WC) and the liquid food product (LP),
determining (206) an ammonium content value (A-r2) that represents the measured ammonium content, and
determining (207) whether the ammonium content value (A-r2) exceeds a threshold (ΔA-n) that is based on a nominal ammonium content value (A-n).

2. The method (200) according to claim 1, wherein the nominal ammonium content value (A-n) is a predetermined value that is associated with the type of food product to which the liquid food product (LP) belongs.

3. The method (200) according to any preceding claim, further comprising
measuring (208), during a first period of time (Δt1), an ammonium content in at least one of the mixture (M), the condensate (WC) and the liquid food product (LP),
determining (209) a set of ammonium content values (A-r1) that represent the ammonium content measured during the first period of time (Δt1), and
setting (210) the nominal ammonium content value (A-n) as a mean of the determined ammonium content values (A-r1).

4. The method (200) according to claim 3, wherein the set nominal ammonium content value (A-n) is, during a second period of time (Δt2) that is subsequent to the first period of time (Δt1), used for the determining (207) of whether the ammonium content value (A-r2) exceeds the threshold (ΔA-n) .

5. The method (200) according to any preceding claim, wherein
the measuring (206) of the ammonium content (A-r1) comprises measuring the ammonium content (A-r1) during a period of time,
the determining (207) of the ammonium content value (A-r2) comprises setting the ammonium content value (A-r2) as a mean of measured (206) ammonium contents (A-r1) during said period of time.

6. The method (200) according to claim 5, further comprising
cleaning at least one component that is used for the producing of the liquid food product (LP), and, in response to said cleaning,
measuring the ammonium content (A-r1) during a further period of time, and
setting the ammonium content value (A-r2) as a mean of the measured ammonium contents (A-r1) during said further period of time.

7. The method (200) according to any preceding claim, wherein the threshold (ΔA-n) comprises a range that is offset from the nominal ammonium content value (A-n) by at least 10%.

8. The method (200) according to any preceding claim, wherein the mixing (201) and the heat treating (202) is performed to produce an ammonium content in the liquid food product (LP) that is
in the range from 3 to 150 mg/l,
preferably in the range from 25 to 150 mg/l when the food material (F) is oat, and
preferably in the range from 3 to 15 mg/l when the food material (F) is soy.

9. The method (200) according to any of the preceding claims, wherein the
the condensing (204) is performed by using a condenser (16), and
the measuring (205) of the ammonium content in the condensate (WC) is performed by using a sensor (118) positioned in or downstream the condenser (16).

10. The method (200) according to claim 9, wherein the sensor (118) is a sensor comprising ion-selective electrodes.

11. The method (200) according to any preceding claim, wherein the measuring (205) of an ammonium content comprises measuring the ammonium content in the condensate (WC).

12. The method (200) according to any preceding claims, further comprising hydrolyzing (211) the food material in the mixture (M) with at least one enzyme (E).

13. The method (200) according to claim 12, wherein the at least one enzyme (E) is one or both of amylase and protease.

14. The method (200) according to any preceding claims, wherein the food material (M) is a plant-based material, preferably oat, whey, rice, soy, or spelt.

15. A system (1, 1') for producing a liquid food product (LP), the system (1. 1') comprising
a mixing arrangement (6) configured to mix water (W) and a food material (F) to provide a mixture (M),
a heat treatment arrangement (12,128,18) configured to heat treat the mixture (M) for deactivating harmful microorganisms,
a flash vessel (14) or an evaporator (18) configured to evaporate water from the mixture (M), to provide a stream of water vapor (WV), and a stream of liquid that forms the liquid food product (LP),
a condenser (16) configured to condense the water vapour (WV) to form a condensate (WC),
a control unit (129) comprising software instructions for
measuring an ammonium content (A-r1) in at least one of the mixture (M), the condensate (WC) and the liquid food product (LP),
determining an ammonium content value (A-r2) that represents the measured ammonium content, and
determining whether the ammonium content value (A-r2) exceeds a threshold (ΔA-n) that is based on a nominal ammonium content value (An).
